# EUROPEAN PATENT APPLICATION

(11) **EP 4 289 382 A1**
(43) Date of publication of application: **13.12.2023**
(21) Application number: 22860249.6
(22) Date of filing: 10.08.2022
(51) Int. Cl.: A61B 34/20

(54) **METHOD FOR ACCURATELY POSITIONING NAVIGATION TARGET POINT**

(30) Priority: 25.08.2021 CN 202110985093
(71) Applicant: Accu Target Medipharma (Shanghai) Co., Ltd., Shanghai 200000 (CN)
(72) Inventor: FEI, Dai, Shanghai 201318 (CN); XU, Shunli, Shanghai 201318 (CN); GONG, Xiaopeng, Shanghai 201318 (CN); WANG, Yu, Shanghai 201318 (CN); YAO, Fengjie, Shanghai 201318 (CN)
(74) Representative: Argyma
(86) International application number: PCT/CN2022/111436
(87) International publication number: WO 2023/024903

(57) **Abstract**

The invention relates to a method for accurately positioning a navigation target point, which includes: selecting a navigation reference with a fixed position, acquiring positions of the navigation reference and the navigation target point in a navigation coordinate system respectively, and calculating a position of the navigation target point relative to the navigation reference, so as to further obtain a position of the navigation target point in a coordinate system of the navigation reference. In the invention, as long as the navigation reference does not move, the coordinate system of the navigation reference will not change during the entire navigation process, and spatial position coordinates of the navigation target point in the coordinate system of the navigation reference will not be interfered by the navigation coordinate system.

## Description

### BACKGROUND OF THE INVENTION

### Field of the Invention

The invention relates to the technical field of computer-aided surgical navigation, in particular, to a method for accurately positioning a navigation target point.

### Description of the Prior Art

In common thoracoabdominal surgery, the chief surgeon usually cuts the patient's chest and abdomen completely open to intuitively perceive the position, size, shape, hardness, and other information of the lesion to be treated by means of naked eyes or touch, thereby making direct surgical treatment for the corresponding lesion.

This is not the case with minimally invasive surgery. In common minimally invasive surgery, the chief surgeon does not completely cut the patient's chest and abdomen to directly face the lesion, but indirectly observes the corresponding lesion through auxiliary means such as laparoscopy, CT, ultrasound, and nuclear magnetic resonance, and then employs some specific means to treat the lesions so as to achieve the purpose of reducing surgical pains, reducing postoperative complications, and accelerating healing of surgical wounds.

However, the minimally invasive surgery also has its disadvantages, wherein the biggest disadvantage is the indirectness of acquiring the lesion information. Therefore, there are various limitations when the chief surgeon acquires the information of the corresponding lesion during the surgery. Taking thoracoabdominal tumor ablation as an example, the chief surgeon needs to perform ablation after puncturing the corresponding ablation needle to the designated location of the lesion. Since the corresponding lesions are hidden in the body, they are not directly visible to the chief surgeon in the minimally invasive surgery.

Thus, in many cases, imaging (such as CT, etc.) and navigation positioning methods are combined with auxiliary guidance to help the doctors perform ablation needle puncture. In this process, there must be a step of establishing a mapping between the navigation positioning data and the image data. In common navigation positioning technology, the coordinate system where the navigation information is located is usually established based on the position of the specified navigation hardware (such as the electromagnetic generator in electromagnetic navigation technology, the optical camera in optical navigation technology, etc.). This method has the disadvantages that the mapping accuracy is easily affected by the position of the designated hardware, e.g., the slight vibration of the designated navigation hardware during the navigation process, which will seriously affect the accuracy in the navigation.

### SUMMARY OF THE INVENTION

In order to solve the above problems, the invention provides a method for accurately positioning a navigation target point, which includes: selecting a navigation reference with a fixed position, acquiring positions of the navigation reference and the navigation target point in a navigation coordinate system respectively, and calculating a position of the navigation target point relative to the navigation reference, so as to further obtain a position of the navigation target point in a coordinate system of the navigation reference.

Preferably, the method includes: acquiring, by a spatial observation devices, a real-time position and shape feature information of the navigation reference and a real-time position of the navigation target point, in which both the navigation target point and the navigation reference are respectively provided with a positioning apparatus.

Preferably, the method includes: acquiring, by the spatial observation device, rotation quaternions and spatial position coordinates of the navigation reference in real time in the navigation coordinate system as (Qo, Qx, Qy, Qz, Tx0, Ty0, Tz0) and position coordinates of the navigation target point as (Tx1, Ty1, Tz1);
the method includes: calculating a corresponding rotation matrix M based on the quaternions (Qo, Qx, Qy, Qz);
multiplying a set of spatial position coordinates (Tx0, Ty0, Tz0) of the navigation reference and a set of spatial position coordinates (Tx1, Ty1, Tz1) of the navigation target point in the navigation coordinate system by the rotation matrix M respectively to obtain the spatial position coordinates of the navigation reference (Tx01, Ty01, Tz01) and the spatial position coordinates of the navigation target point (Tx11, Ty11, Tz11) in a temporary coordinate system, wherein a coordinate origin of the temporary coordinate system coincides with a origin of the navigation coordinate system, and directions of coordinate axes of the temporary coordinate system are consistent with shape features of the navigation reference;
calculating position coordinates of the navigation target point in a coordinate system of the navigation reference as (Tx11-Tx01, Ty11-Ty01, Tz11-Tz01) based on the position parameters of the navigation reference (Tx01, Ty01, Tz01) and the position parameters of the navigation target point (Tx11, Ty11, Tz11) in the temporary coordinate system.

Preferably, the navigation reference is fixed at a place where a body surface of a patient does not move or other places fixed relative to the a body of the patient.

Preferably, a position of the origin and directions of the coordinate axes of the navigation coordinate system are determined by navigation hardware.

Preferably, the navigation hardware is an electromagnetic generator in electromagnetic navigation technology or an optical camera in optical navigation technology.

Preferably, the method is applied in thoracoabdominal puncture surgery.

Preferably, the navigation target point is a puncture needle.

Preferably, a shape of the navigation reference is a cross.

Compared with the prior art, the invention has the following technical effects:
In normal navigation surgery such as navigation puncture surgery, due to various unavoidable factors such as human touch by mistake or slight vibration, the navigation hardware often has some imperceptible deviations. Since the origins and the directions of the coordinate axes of the navigation coordinate system are usually related to the corresponding navigation hardware (as the saying goes, a slight mistake is a thousand miles away), these deviations usually easily lead to large errors in the navigation position, which will affect the navigation accuracy, thereby increasing the difficulty of the surgery for the doctors and increasing the risk of the patient. Using the method will be able to avoid this problem, not only reduce the difficulty in surgery for the doctors, but also improve the accuracy in surgery, and reduce the risk of the surgery, thereby protecting the patient.

### BRIEF DESCRIPTION OF THE DRAWINGS

In order to more clearly describe the technical solutions of the embodiments of the present invention, the accompanying drawings required to describe the embodiments are briefly described below. Apparently, the accompanying drawings described below are only some embodiments of the present invention. A person skilled in the art may obtain other drawings from the accompanying drawings without involving an inventive effort. In the drawings:
Fig. 1 is a comparison chart showing the changes before and after the navigation coordinate system is disturbed;
Fig. 2 is a diagram showing that the introduction of the coordinate system of the navigation reference to observe the navigation target point according to the invention may ensure that the readings of positions and directions of the navigation target point will not change due to the changes of the navigation coordinate system;
Fig. 3 is a diagram showing the calculation steps of the positions of the navigation target point in the coordinate system of the navigation reference according to the invention;
Fig. 4 is a flow chart of calculating the positions of the navigation target point in the coordinate system of the navigation reference according to the invention;
Fig. 5 is a diagram showing the appearance of the navigation reference and the corresponding coordinate axes according to the invention.

### DESCRIPTION OF THE PREFERRED EMBODIMENTS

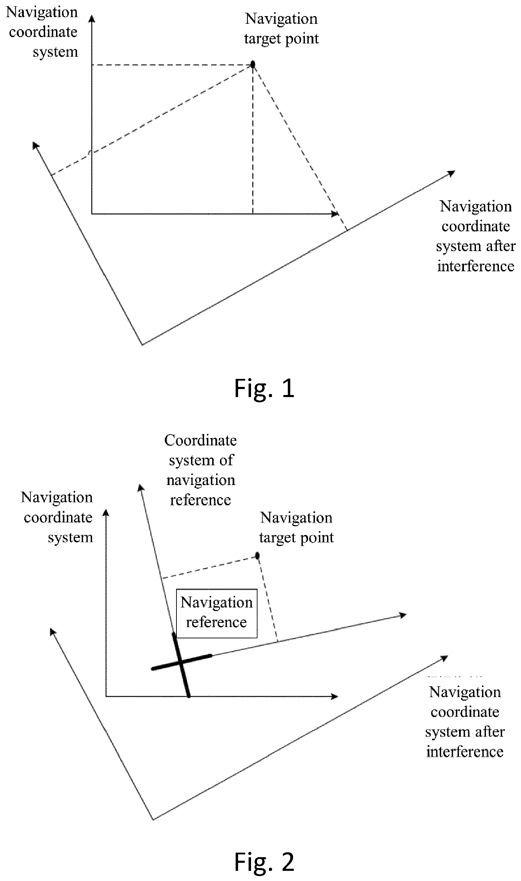

In thoracoabdominal puncture surgery guided by CT imaging based on navigation technology, the position of the origin and the directions of the corresponding coordinate axes of the navigation coordinate system are usually determined by specified navigation hardware (such as electromagnetic generators in electromagnetic navigation technology, optical cameras in optical navigation technology, etc.). However, in the process of surgery, due to some factors (such as accidental touch by people, slight vibration of navigation hardware caused by various factors, etc.), usually the positions and the orientation of the corresponding navigation hardware will change, so that the corresponding navigation coordinate system will also change accordingly, and the readings of the positions of the navigation target point based on the navigation coordinate system will also change significantly, as shown in Fig. 1.

If there is a navigation reference at a fixed position, which is fixed at a certain position, and if it is ensured that the positions and the orientation will not be affected by various factors (such as accidental touch by people mentioned above, slight vibration of hardware caused by various factors, etc.) while ensuring that the positions and the orientation have nothing to do with the navigation hardware that determines the navigation coordinate system while the positions and shape features may be observed and identified by a surgical navigation system, then the positions and shape directions of the navigation reference in the navigation coordinate system may be calculated based on the above and a coordinate system of the navigation reference may be established based on the above, so that it may be ensured that the readings of the positions and the directions of the navigation target point will not change due to the changes of the navigation coordinate system when the coordinate system observes the navigation target point, as shown in Fig. 2.

With reference to Fig. 2, after the navigation coordinate system changes, the coordinate positions of the navigation reference in the navigation coordinate system will also change, and the coordinate positions of the navigation target point in the navigation coordinate system will also change. However, the relative position of the navigation target point relative to the navigation reference calculated based on the coordinate position data of the navigation reference and the navigation target point in the navigation coordinate system will not change. Therefore, it can be believed that as long as the navigation reference does not move, the coordinate system of the navigation reference will not change during the entire navigation process, spatial position coordinates (position parameters) of the navigation target point in the coordinate system of the navigation reference will not be interfered by the navigation coordinate system, and the position (the position parameter of the navigation target point in the coordinate system of the navigation reference) has nothing to do with the navigation coordinate system.

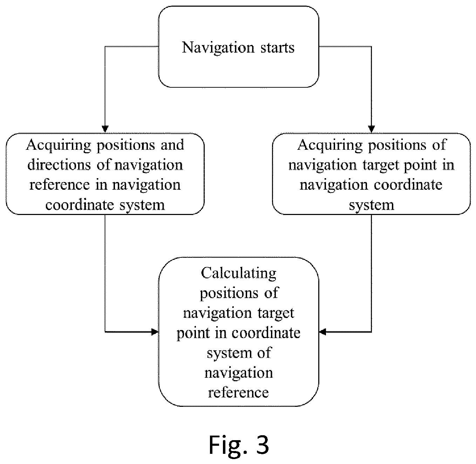

In summary, in order to avoid the large deviation of the coordinate readings of the navigation target point due to the slight vibration of the navigation hardware, with reference to Fig. 3, the invention provides a method for accurately positioning a navigation target point, which includes: selecting a navigation reference with a fixed position, acquiring positions of the navigation reference and the navigation target point in a navigation coordinate system respectively, and calculating a position of the navigation target point relative to the navigation reference after the navigation starts, so as to further obtain a position of the navigation target point in a coordinate system of the navigation reference.

The method for accurately positioning a navigation target point provided by the invention is based on the surgical navigation system and may be widely used in various surgical operations such as neurosurgery, spinal neurosurgery, etc., and also applied in minimally invasive operations such as thoracoabdominal puncture, tissue biopsy puncture, etc. Detailed descriptions will be made below on the method for accurately positioning a navigation target point provided by the invention applied to the thoracoabdominal puncture surgery guided by CT imaging based on the navigation technology (including optical navigation or electromagnetic navigation) in combination of Figs. 1 to 3. The embodiment is implemented under the premise of the technical solution of the invention, and detailed implementation and specific operation process are provided. However, the scope of protection of the invention is not limited to the following embodiments, and those skilled in the art can modify and embellish it within the scope of not changing the spirit and content of the invention.

In the embodiment, when the method positions a monitored target, a spatial position observation device (short for a spatial observation device), two positioning apparatuses corresponding to the spatial observation device, a navigation reference with obvious shape features (the invention does not specifically limit the shape of navigation reference, and the embodiment takes a cross shape as an example) and a corresponding puncture needle (the invention does not limit the specific type of the target point, which may be determined according to the specific operation, and the embodiment is used in thoracoabdominal puncture surgery; therefore, the navigation target point is the puncture needle) must be used, wherein the two positioning apparatuses are respectively provided on the navigation reference and the puncture needle, and the shape features and precise real-time positions of the navigation reference and the precise real-time positions of the puncture needle may be tracked by the spatial position observation device.

The method has steps, which may be roughly divided into several stages: a stage of installing observation apparatuses, a stage of tracking with the navigation apparatus, and a stage of conversion for observed targets:

### Stage of installing observation apparatuses

In the stage of installing observation apparatuses, the doctor must install a positioning apparatus on the navigation reference, and ensures that during the entire navigation process, the observation device may accurately acquire the real-time position and the shape feature information of the navigation reference. At the same time, the navigation reference is pasted on a body surface of a patient or fixed to other positions that are relatively fixed relative to a body of the patient and easy to be observed by the observation device, and it must be ensured that the position will not be affected by factors such as breathing and is relatively static to the body.

### Stage of tracking with the navigation apparatus

In the stage of tracking with the navigation apparatus, the spatial observation device may acquire the real-time position and the shape feature information of the navigation reference in real-time, which are comprehensively expressed as rotation quaternions and spatial position coordinates (Qo, Qx, Qy, Qz, Tx0, Ty0, Tz0) in the navigation coordinate system, wherein the first 4 parameters are rotation quaternions, and the last 3 parameters are three-dimensional spatial position coordinates. Meanwhile, the spatial observation device may further acquire the position-related parameters (Tx1, Ty1, Tz1) of the navigation target point in real time.

### Stage of conversion for observed targets

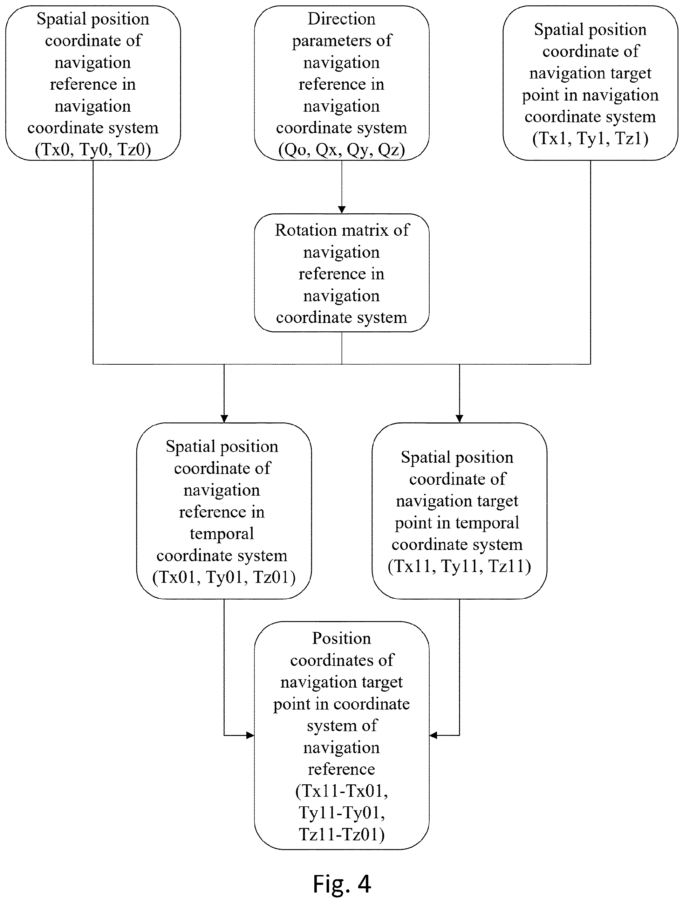

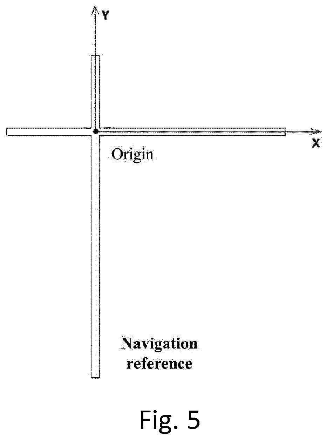

After the rotation quaternions and spatial position coordinates (Qo, Qx, Qy, Qz, Tx0, Ty0, Tz0) in the navigation coordinate system and the position-related parameters (Tx1, Ty1, Tz1) of the navigation target point are acquired, positions of the navigation target point in the coordinate system of the navigation reference may be calculated through a series of mathematical operations based on these two sets of parameters. The calculation process is shown in Fig. 4:
1. A corresponding rotation matrix M is calculated based on the direction parameters (Qo, Qx, Qy, Qz) of the navigation reference;
2. The spatial position coordinates (Tx0, Ty0, Tz0) of the navigation reference and the spatial position coordinates (Tx1, Ty1, Tz1) of the navigation target point in the navigation coordinate system are multiplied by the rotation matrix M respectively to obtain the spatial position coordinates of the navigation reference (Tx01, Ty01, Tz01) and the spatial position coordinates of the navigation target point (Tx11, Ty11, Tz11) in a temporary coordinate system. It should be noted that a coordinate origin of the temporary coordinate system coincides with the navigation coordinate system, and directions of coordinate axes of the temporary coordinate system are consistent with the shape features of the navigation reference. With reference to Fig. 5, the conversion method for the navigation coordinate system and the coordinate system of the navigation reference is performed by first keeping the origin of the navigation coordinate system fixed, rotating the navigation coordinate system so that the directions of the corresponding coordinate axes are parallel to the coordinate system of the navigation reference, then moving the position of the origin to the origin of the coordinate system of the navigation reference so that the origin of the coordinate system of the navigation reference is an intersection point of the cross-shaped navigation reference. The temporary coordinate system here refers to an intermediate state in the conversion process. The origin of the coordinate system coincides with the navigation coordinate system, but the directions of the coordinate axes are consistent with the coordinate system of the navigation reference.
3. The position coordinates of the navigation target point in a coordinate system of the navigation reference are calculated as (Tx11-Tx01, Ty11-Ty01, Tz11-Tz01) based on two sets of spatial position coordinates (Tx01, Ty01, Tz01) and (Tx11, Ty11, Tz11) in the temporary coordinate system.

Due to technical reasons, there is no way to directly generate or use the coordinate system of the navigation reference; only after the position data of the navigation reference and the navigation target point in the navigation coordinate system are acquired, the relative position of the navigation target point and the navigation reference is calculated, and then the position data of the navigation target point in the coordinate system of the navigation reference are calculated. Therefore, in the embodiment, it is not necessary to calculate the coordinate system of the navigation reference itself, but to calculate the coordinate position of the navigation target point in the coordinate system of the navigation reference.

After the position of the navigation target point in the coordinate system of the navigation reference is calculated, the position may be used in the navigation operation, such as mapping to the navigation image or used to monitor the position of the navigation puncture.

## Claims

1. A method for accurately positioning a navigation target point, the method comprising:
selecting a navigation reference with a fixed position, acquiring positions of the navigation reference and the navigation target point in a navigation coordinate system respectively, and calculating a position of the navigation target point relative to the navigation reference, so as to further obtain a position of the navigation target point in a coordinate system of the navigation reference;
acquiring, by a spatial observation device, a real-time position and shape feature information of the navigation reference and a real-time position of the navigation target point, wherein both the navigation target point and the navigation reference are respectively provided with a positioning apparatus;
acquiring, by the spatial observation device, rotation quaternions and spatial position coordinates of the navigation reference in real time in the navigation coordinate system as (Qo, Qx, Qy, Qz, Tx0, Ty0, Tz0) and position coordinates of the navigation target point as (Tx1, Ty1, Tz1);
calculating a corresponding rotation matrix M based on the quaternions (Qo, Qx, Qy, Qz);
multiplying a set of spatial position coordinates (Tx0, Ty0, Tz0) of the navigation reference and a set of spatial position coordinates (Tx1, Ty1, Tz1) of the navigation target point in the navigation coordinate system by the rotation matrix M, respectively, to obtain the spatial position coordinates of the navigation reference (Tx01, Ty01, Tz01) and the spatial position coordinates of the navigation target point (Tx11, Ty11, Tz11) in a temporary coordinate system, wherein a coordinate origin of the temporary coordinate system coincides with a origin of the navigation coordinate system, and directions of coordinate axes of the temporary coordinate system are consistent with shape features of the navigation reference; and
calculating position coordinates of the navigation target point in a coordinate system of the navigation reference as (Tx11-Tx01, Ty11-Ty01, Tz11- Tz01) based on the spatial position coordinates of the navigation reference (Tx01, Ty01, Tz01) and the spatial position coordinates of the navigation target point (Tx11, Ty11, Tz11) in the temporary coordinate system.

2. The method for accurately positioning a navigation target point according to claim 1, wherein the navigation reference is fixed at a place where a body surface of a patient does not move or other places fixed relative to the a body of the patient.

3. The method for accurately positioning a navigation target point according to claim 1, wherein a position of the origin the navigation coordinate system and the directions of the coordinate axes of the navigation coordinate system are determined by navigation hardware.

4. The method for accurately positioning a navigation target point according to claim 3, wherein the navigation hardware is an electromagnetic generator in electromagnetic navigation technology or an optical camera in optical navigation technology.

5. The method for accurately positioning a navigation target point according to any one of claims 1 to 4, applied in thoracoabdominal puncture surgery.

6. The method for accurately positioning a navigation target point according to claim 5, wherein the navigation target point is a puncture needle.

7. The method for accurately positioning a navigation target point according to claim 1, wherein a shape of the navigation reference is a cross.
